# EUROPÄISCHE PATENTSCHRIFT

(11) **EP 3 378 628 B1**
(45) Veröffentlichungstag und Bekanntmachung des Hinweises auf die Patenterteilung: **04.01.2023**
(21) Anmeldenummer: 18163176.3
(22) Anmeldetag: 21.03.2018
(51) Int. Cl.: B29C 64/165, A61C 13/00, B28B 1/00, B29C 64/153, B29C 64/112

(54) **VERFAHREN ZUR GENERATIVEN HERSTELLUNG EINES DENTALEN FORMKÖRPERS**
METHOD FOR GENERATIVE PRODUCTION OF A DENTAL MOULD
PROCÉDÉ DE FABRICATION GÉNÉRATIVE D'UN CORPS MOULÉ DENTAIRE

(30) Priorität: 21.03.2017 DE 102017106101
(43) Veröffentlichungstag der Anmeldung: 26.09.2018
(73) Patentinhaber: decema GmbH, 78247 Hilzingen (DE)
(72) Erfinder: Köbel, Stefan, 8447 Dachsen (CH)
(74) Vertreter: Molnia, David

(56) Entgegenhaltungen:
- WO-A1-2014/067990
- DE-A1-102011 117 005
- DE-T2- 69 025 147
- US-B1- 6 322 728

## Beschreibung

### Technisches Gebiet

Die vorliegende Erfindung betrifft ein Verfahren zur generativen Herstellung eines dentalen Formkörpers.

### Stand der Technik

Additive Fertigungsverfahren ermöglichen die Herstellung von Formkörpern aus Keramiken und/oder pulvermetallurgischen Werkstoffen aus dreidimensionalen Computermodellen, wie beispielsweise CAD-Daten, ohne die Erfordernisse von Bauteil-spezifischen Werkzeugen. Bei additiven Fertigungsverfahren werden abwechselnd Schichten einer Dispersion und eines Härters auf einen Hilfsträger aufgebracht. Für das Aufbringen der Schichten auf den Hilfsträger kommen insbesondere Druckverfahren und/oder Sprühverfahren zum Einsatz. Bei der Härtung werden beispielsweise Effekte von Polymerisation ausgenutzt. Durch das wiederholte Übereinanderdrucken der Schichten entsteht ein Rohkörper als dreidimensionales Objekt. Da die aufwendige Erzeugung von Vergusskörpern entfällt eignen sich additive Fertigungsverfahren insbesondere für die Bereitstellung von dentalen Prothesen, wie Zahnimplantaten, -inlays, -kronen und -brücken.

Im Gegensatz zu Laserschmelzen und Binder-Jetting, bei denen der zu verarbeitende Rohstoff jeweils in Pulverform auf einen Hilfsträger aufgebracht wird, kann mit einem additiven Fertigungsverfahren eine größere Auswahl an Materialien verarbeitet werden, da das Einbringen des Materials in Pulverform in eine Dispersion höhere Dichten und eine höhere Homogenität des Rohkörpers ermöglicht. Zudem werden, anders als bei der sogenannten Stereolithographie, keine Monomere, die ein Risiko für Gesundheit und Umwelt darstellen, benötigt und der Gesamtpolymergehalt ist signifikant niedriger, wodurch nachfolgende Entbinderungs- und Brennprozesse des Bauteils erleichtert werden.

Bisher bekannte additive Fertigungsverfahren haben jedoch den Nachteil, dass für eine effiziente Herstellung der gewünschten Rohkörper oftmals gesundheitlich bedenkliche Chemikalien zum Einsatz kommen, oder gesundheitlich bedenkliche Stäube auftreten können. Ferner ist die Bereitstellung sogenannter Gradientenbauteile, deren physikalische und/oder chemische Eigenschaften sich über das Volumen des Bauteils verändern, sehr aufwendig und in gewissen technischen Bereichen nicht möglich. Insbesondere sind keine additiven Verfahren für eine individuelle Anpassung physikalischer und/oder chemischer Eigenschaften entlang des Rohkörpers bekannt.

Aus der WO 2014/067990 A1 ist ein generatives Verfahren zur Herstellung eines keramischen Grün- beziehungsweise Formkörpers bekannt. Insbesondere wird auf ein keramisches, glaskeramisches oder Glaspulver eine Verfestigungszusammensetzung appliziert, welche eine Organoelementverbindung, einen organischen Binder und ein Lösungs- oder Dispergiermittel enthält. Das Verfahren reduziert das Verstopfen von Druckköpfen und ermöglicht ein wesentlich homogenes Farbbild. Einen Nachteil stellt jedoch die verhältnismäßig komplexe Zusammensetzung der Verfestigungszusammensetzung dar.

Die WO 2015/168463 A1 zeigt ein Verfahren zur Herstellung von Zahnersatz. In einem ersten Schritt wird ein Pulverbett bereitgestellt, welches in einem zweiten Schritt selektiv mit einem Binder beaufschlagt wird. Die Schritte werden mehrfach wiederholt, bis der gewünschte Grünkörper entstanden ist. Schließlich wird der nicht mit Binder versehene Teil des Pulvers entfernt und der Grünkörper gesintert. Das Verfahren birgt den Nachteil, dass das Pulverbett vergleichsweise große Korngrößen im 10er Mikrometerbereich voraussetzt. Dies führt zu vergleichsweise großen Zwischenräumen zwischen den einzelnen Körnern, welche sich nachteilig auf den Sinterprozess sowie die physikalischen und ästhetischen Eigenschaften des fertigen Formkörpers auswirken können.

Die Druckschrift DE 10 2011 117 A1 zeigt ein Verfahren zur Herstellung eines keramischen Formkörpers, insbesondere in Form eines Zahnersatzes, auf Basis eines generativen Herstellungsverfahrens. Dabei werden einzelne Schlickerschichten jeweils ortsselektiv mit Farbpigmenten und einer die Schlickerschicht verfestigenden Stoffzusammensetzung beaufschlagt. Schließlich wird der Grünkörper zum Erhalt eines Formkörpers gesintert. Ein Nachteil an diesem Verfahren, stellt die Notwendigkeit der zusätzlichen Bereitstellung von Farbpigmenten zur Farbgebung des Formkörpers dar.

US 6 322 728 B1 zeigt die Massenproduktion von Zahnersatz durch feste Freiformfertigungsmethoden. DE 690 25 14 7 T2 zeigt dreidimensionale Drucktechniken.

### Darstellung der Erfindung

Ausgehend von dem bekannten Stand der Technik ist es eine Aufgabe der vorliegenden Erfindung, ein verbessertes Verfahren zur generativen Herstellung eines Formkörpers bereitzustellen. Insbesondere soll die Möglichkeit der Einstellung der Farbgebung und anderer physikalischer Eigenschaften verbessert werden.

Die Aufgabe wird durch ein Verfahren gemäß dem unabhängigen Anspruch 1 gelöst. Vorteilhafte Weiterbildungen ergeben sich aus den abhängigen Ansprüchen, der Beschreibung sowie den Ausführungsbeispielen. Entsprechend wird ein Verfahren zur generativen Herstellung dentaler Formkörper für die Zahnrestauration und/oder als Zahnersatz angegeben. Das Verfahren umfasst die folgenden Schritte: a) Bereitstellen zumindest einer Schicht einer Dispersion, insbesondere einer wässrigen Dispersion, wobei die Dispersion mindestens einen Binder aufweist, und wobei die Dispersion Keramikpartikel und/oder Glaskeramikpartikel und/oder Pulvermetallpartikel aufweist; b) stellenweises Aufbringen von Härtern auf die Schicht der Dispersion zur Aktivierung des mindestens einen Binders zur stellenweisen Verfestigung der Schicht der Dispersion, wodurch ein Rohkörper erhalten wird, insbesondere ein Grünkörper, wobei mindestens zwei Härter mit voneinander unterschiedlicher Materialzusammensetzung verwendet werden; und c) Sintern des Rohkörpers zu dem dentalen Formkörper.

Unter dem Begriff Härter wird eine Substanz verstanden, die durch eine chemische Reaktion mit einem Polymer (oder Monomer) ein dreidimensionales Netzwerk bildet - genau so, wie dies zum Beispiel bei 2-Komponenten Klebstoffen oder beim Gelieren (Sol-Gel Übergang) von Polymeren der Fall ist.

Hydrogel-bildende Polymere können natürlichen oder synthetischen Ursprungs sein. Hydrogele bestehen aus einem dreidimensionalen Polymernetzwerk das eine wässrige Phase stabilisiert. Hydrogele entstehen durch physikalische und/oder chemische Vernetzung. Chemisch vernetzte Hydrogele weisen kovalente Bindungen auf (z. B. Polyacrylamid, Polyethylenglycol (PEG), Hyaluronsäure, Chitosanderivate usw.), während physikalisch vernetzte Hydrogele transiente, reversible Verbindungen aufweisen, die von den Wechselwirkungen zwischen Polymerketten abgeleitet sind, wie dem Verknäulen (z. B. Polyvinylalkohol, Kollagen, Gelatine, vielen Polysacchariden), ionischen Bindungen (z. B. Alginat, Pektin usw.) oder Wasserstoffbrückenbindungen. Hydrogele können auch durch die Modifikation von wasserlöslichen Polymeren gebildet werden, indem reaktive Gruppen in das Polymer eingebaut werden die das Polymer chemische vernetzbar machen oder durch Stimuli wie Licht, Wärme oder pH, oder Kombinationen davon, zur Vernetzung angeregt werden können.

Das hier beschriebene Verfahren zur generativen Herstellung eines Formkörpers vereint konzeptionell die Vorteile der Stereolithographie und des Binder-Jettings. Der Vorteil des hier beschriebenen Verfahrens liegt insbesondere in der Variation des Härters. Zumindest einer der Härter kann zusätzliche Komponenten enthalten, um den Rohkörper während des Druckvorgangs zu Dotieren und somit dem finalen Formkörper zusätzliche Eigenschaften zu verleihen. Durch die Verwendung unterschiedlicher Härter ergibt sich bei dem Herstellungsverfahren ein zusätzlicher Freiheitsgrad hinsichtlich der physikalischen und/oder chemischen Eigenschaften.

Die Härter können insbesondere als Lösung bereitgestellt werden. Beispielsweise enthalten die Härter jeweils de-ionisiertes Wasser, eine Salzlösung, bevorzugt eine höchstens 1-molare Metallsalzlösung und Additive. Die Additive können kationische Verflüssiger, Alkohole und/oder Komplexbildner enthalten. Mittels schnell verdampfender Alkohole kann insbesondere eine Tropfengröße beim Aufbringen der Härter reduziert werden. Eine Reduktion der Tropfengröße ermöglicht eine Verbesserung der Ortsauflösung bei dem Herstellungsverfahren und damit die präzisere Anpassung der Form und/oder Eigenschaften des Rohkörpers beziehungsweise des Formkörpers.

Ferner können keramische Farbkörper und/oder weitere Partikel in die Härter eingebracht sein. Bevorzugt enthält zumindest einer der Härter mehrwertige Ionen, insbesondere Kationen von Übergangsmetallen. Die Ionen können entsprechend der im Formkörper gewünschten Farbgebung und/oder anderer im Formkörper zu erzielender physikalischer und/oder chemischer Eigenschaften gewählt werden. Beispielsweise handelt es sich bei den Ionen um Zirkonium-Ionen. Für spezielle gewünschte Farbtöne können anderweitige Ionen eingebaut werden. So erzeugen Kobalt-Ionen zum Beispiel nach dem Sintern eine Blaufärbung von Aluminiumoxid.

In dem Verfahrensschritt a) wird die Dispersion bevorzugt auf einen Hilfsträger aufgebracht, beispielsweise durch Aufsprühen. Die Dispersion kann eine sogenannte Binder-Dispersion sein oder eine solche Binder-Dispersion enthalten. Beispielsweise enthält die Dispersion de-ionisiertes Wasser, keramische Rohstoffe, Verflüssiger, einen Polyelektrolyten wie zum Beispiel Alginat, insbesondere Ammonium-Alginat, und/oder weitere Additive, wie beispielsweise Glycerol und/oder Zitronensäure. Die keramischen Rohstoffe können zum Beispiel Aluminiumoxid (Al₂O₃) enthalten. Bevorzugt beträgt der Feststoffgehalt der Dispersion wenigstens 40 vol%, besonders bevorzugt wenigstens 50 vol%. Die Feststoffe der Dispersion können in der Form eines Pulvers in die Dispersion eingebracht sein.

Der Binder kann irgendein Polymer sein, das durch Ionen vernetzt werden kann, um ein Gel zu bilden. Bevorzugt werden Metallionen für die Vernetzung verwendet. Besonders bevorzugt sind die Ionen mehrwertige Kationen.

lonotrope hydrogelbildende Biopolymere, Polyelektrolyte und synthetische Polymere können als Binder verwendet werden. Solche Polymere sind dem Fachmann bereits bekannt.

Beispiele für solche Polymere, die unter Verwendung von multivalenten Ionen vernetzt werden können, sind: Polysaccharide, zum Beispiel Karrageen, Dextrin, Gellan, Scleroglucan, Chitosan und dessen Derivate; wasserlöslichen Polyphosphazenen wie poly(bis(4-Carboxyphenoxy)Phosphazen); Polyacrylate; und Polyaminosäuren.

In einer bevorzugten Ausführungsform sind der mindestens eine Binder und die Keramikpartikel und/oder Glaskeramikpartikel und/oder Pulvermetallpartikel in der Dispersion homogen verteilt.

Die Dispersion wird beispielsweise mittels eines Rakels egalisiert und/oder durch Vibrationen homogen auf dem Hilfsträger verteilt. Durch Vibrationen wie beispielsweise ein Hin- und Herbewegen ergibt sich ein homogener beziehungsweise ebener Spiegel der Dispersion auf dem Hilfsträger.

Auf die Dispersion können dann die Härter aufgebracht werden, beispielsweise unter Verwendung eines Druckvorgangs und/oder durch Aufsprühen. Aufgrund der Diffusion dringen die wirksamen Bestandteile der Härter, insbesondere in den Härtern vorhandene Ionen, in die Dispersion ein und können dort zum lokalen Gelieren der Dispersion führen. Dies entspricht in etwa dem Effekt der Radikale auf die Monomere bei der Stereolithographie.

Bevorzugt wird der Verfahrensschritt a) zum Herstellen des Rohkörpers mehrfach wiederholt. Beispielsweise wird hierfür zunächst eine erste Schicht der Dispersion auf den Hilfsträger aufgebracht. Auf die erste Schicht der Dispersion werden dann Härter aufgebracht. Anschließend kann eine weitere Schicht der Dispersion, oder, einer sich von der Dispersion unterscheidenden weiteren Dispersion auf die verfestigte erste Schicht aufgebracht werden. Die weitere Schicht kann dann ebenfalls unter Verwendung von Härtern verfestigt werden. Als letzter Schritt wird jeweils die Dispersion aufgebracht (sogenanntes "re-coating"). Die Zusammensetzung der Härter, also ihr relativer Anteil, kann sich dabei für die Verfestigung der weiteren Schicht von der Zusammensetzung der Härter, die für die Verfestigung der ersten Schicht verwendet wurden, unterscheiden. Durch das Wiederholen des Verfahrensschritts a) kann so schichtweise ein Rohkörper (sogenannter Grünkörper) bereitgestellt werden.

In einer weiter bevorzugten Ausführungsform des Verfahrens wird ein relativer Anteil der Härter zueinander ortsabhängig gesteuert. Insbesondere kann der Anteil jedes Härters ortsabhängig angepasst werden. Der Begriff "ortsabhängig" beinhaltet hierbei und im Folgenden sowohl eine Anpassung des relativen Anteils in horizontaler beziehungsweise lateraler Richtung als auch in vertikaler Richtung, also in Wachstumsrichtung. Bei dem relativen Anteil eines Härters handelt es sich hierbei und im Folgenden um den Gewichtsanteil oder den Volumenanteil. Durch die ortsabhängige Steuerung des Anteils ist es möglich, physikalische und/oder chemische Eigenschaften des Formkörpers ortsabhängig einzustellen. Insbesondere können unterschiedliche Bereiche des Formkörpers an unterschiedliche Einsatzbereiche angepasst werden.

Gemäß zumindest einer Ausführungsform des Verfahrens enthält zumindest einer der Härter Metallionen. Die Metallionen werden bevorzugt so gewählt, dass der Härter neben der Aushärtung auch zusätzliche Funktionen, wie beispielsweise eine Farbgebung, erzielt. Da für eine gewünschte Farbgebung oftmals Konzentrationen höherer Art benötigt werden, können in diesem Fall ein Teil der Ionen im Härter komplexiert werden.

Es ist insbesondere möglich, dass zumindest einer der Härter Ionen einer ersten Art und/oder einer zweiten Art enthält. Ionen einer weiteren Art, wie beispielsweise einer dritten oder vierten Art, sind ebenfalls möglich. Unterschiedliche Härter können insbesondere Ionen unterschiedlicher Art und/oder unterschiedliche Zusammensetzungen der Ionen unterschiedlicher Art enthalten. Wenn hierbei und im Folgenden allgemein "Ionen" beschrieben sind, sind darunter die Ionen erster Art, die Ionen zweiter Art und/oder Ionen einer weiteren Art zu verstehen. Beispielsweise können als Ionen unterschiedlicher Art homologe Ionen verwendet werden.

Gemäß dem erfindungsgemäßen Verfahren enthält zumindest einer der Härter Ionen zumindest einer ersten Art. Die Reaktionstemperatur der Ionen der ersten Art mit dem pulverförmigen Material liegt unter einer Sintertemperatur, die während des Sinterns in dem Verfahrensschritt b) verwendet wird. Bevorzugt liegt die Reaktionstemperatur von gegebenenfalls vorhandenen Ionen einer zweiten Art und/oder einer weiteren Art ebenfalls unterhalb der Sintertemperatur. Die Sintertemperatur beträgt beispielsweise wenigstens 1000 °C und höchstens 2000 °C, bevorzugt wenigstens 1200 °C und höchstens 1600 °C. Hierdurch ist es möglich, dass die Ionen der ersten Art durch das Sintern nicht aus dem Rohkörper verdampft werden. Während des Sinterns sind die Ionen der ersten Art innerhalb des Kristallgitters des Rohkörpers frei beweglich. Mit anderen Worten, die Ionen der ersten Art lösen sich während des Sinterns zumindest teilweise im Kristallgitter des Rohkörpers in fester Lösung. Hierdurch kann zumindest ein Teil der Ionen der ersten Art innerhalb des Rohkörpers gelöst werden.

Hierbei und im Folgenden allgemein stellen die Begriffe "Ionen erster Art", "Ionen zweiter Art" und "Ionen einer weiteren Art" auf das den Ionen zugrundeliegende chemische Element ab, welches jeweils eines aus der Gruppe Titan, Chrom, Mangan, Eisen, Kobalt, Nickel, Kupfer, Zink, Aluminium, Strontium, Yttrium, Zirkonium, Niob, Lanthan, Cer, Praseodym, Terbium, Erbium, Ytterbium enthalten oder sein kann. Die "Ionen erster Art", "Ionen zweiter Art" und "Ionen einer weiteren Art" entsprechen jeweils einem anderen Element der vorstehenden Gruppe.

Gemäß zumindest einer Ausführungsform des Verfahrens enthält mindestens einer der Härter Ionen zumindest einer zweiten Art. Die Ionen zweiter Art sind während des Sinterns in Verfahrensschritt b) zumindest teilweise in dem Kristallgitter des Rohkörpers in fester Lösung gelöst. Mit anderen Worten, die Ionen zweiter Art sind zumindest teilweise in dem Kristallgitter des Rohkörpers eingebaut und besetzen teilweise Gitterplätze dieses Kristallgitters. Mit anderen Worten, die Ionen zweiter Art können als Dotierung wirken, wodurch physikalische und/oder chemische Eigenschaften des Formkörpers lokal variiert werden können.

Gemäß zumindest einer Ausführungsform des Verfahrens wird die für das Sintern in Verfahrensschritt b) verwendete Sintertemperatur durch die Ionen zweiter Art reduziert. Mit anderen Worten, das Sintern erfordert aufgrund der Ionen zweiter Art eine geringere Sintertemperatur. Die erforderliche Sintertemperatur kann insbesondere proportional zur Solidustemperatur des Materials des Rohkörpers sein. Durch das Einbauen der Ionen zweiter Art während des Sintervorgangs in das Kristallgitter des Rohkörpers kann die Kristallstruktur zumindest lokal verändert werden und die Sintertemperatur reduziert werden. Hierbei und im Folgenden handelt es sich bei der "Kristallstruktur" insbesondere um das Kristallgitter des als Keramik, Glaskeramik und/oder Pulvermetalls vorliegenden Rohkörpers.

Gemäß zumindest einer alternativen oder zusätzlichen Ausführungsform des Verfahrens verändern die Ionen zweiter Art lokal zumindest eine physikalische Eigenschaft und/oder zumindest eine chemische Eigenschaft des gesinterten Formkörpers. Die Anpassung der physikalischen und/oder chemischen Eigenschaften kann insbesondere in einer lokalen Änderung der Kristallstruktur durch die Ionen zweiter Art begründet sein. Lokale Änderungen können sich von globalen Änderungen dadurch unterscheiden, dass lediglich ein Teilbereich der Kristallstruktur des Rohkörpers variiert wird. Beispielsweise werden der Gitterabstand und/oder die Bindungsenergie der Kristallstruktur durch die Ionen zweiter Art geändert.

Bei der physikalischen Eigenschaft handelt es sich bevorzugt um eine optische Eigenschaft des Formkörpers. Ferner handelt es sich bei der chemischen Eigenschaft bevorzugt um eine katalytische Eigenschaft des Formkörpers. Optische Eigenschaften des Formkörpers sind beispielsweise dessen Farbe, Transparenz, Opazität und/oder Brechungsindex. Die Änderung der optischen Eigenschaften kann einerseits aus einem Einbauen von optisch aktiven Ionen in die Kristallstruktur des Rohkörpers und/oder aus einer zumindest lokalen Veränderung der Bandlücke des Kristallgitters des Formkörpers resultieren.

Erfindungsgemäß enthält mindestens einer der Härter Ionen. Bei den Ionen kann es sich um die Ionen erster Art, die Ionen zweiter Art und/oder gegebenenfalls vorhandene Ionen einer weiteren Art handeln. Die Ionen, insbesondere die Ionen erster Art und/oder die Ionen zweiter Art, bilden während des Sinterns in Verfahrensschritt b) Ausscheidungen im Gefüge der Kristallstruktur des Rohkörpers. Der aus dem Rohkörper entstandene Formkörper kann dann ein anisotropes Gefüge, insbesondere ein Zweiphasengefüge, aufweisen, das sich während dem Sintern einstellt. Es ist insbesondere möglich, die Position der Ausscheidungen durch die Wahl der Ionen und/oder der Position der Ionen vor dem Sintern zu steuern und so einen Anteil der Ionen lokal entlang des Rohkörpers zu variieren.

Gemäß zumindest einer Ausführungsform des Verfahrens sind die Ionen des zumindest einen Härters, insbesondere die Ionen erster Art und/oder die Ionen zweiter Art, Kationen mit einer Wertigkeit von wenigstens drei. Die Kationen können also insbesondere dreiwertig (trivalent) oder höherwertig, wie beispielsweise vierwertig (tetravalent), sein.

Gemäß zumindest einer Ausführungsform des Verfahrens enthält die Dispersion keramische und/oder glaskeramische und/oder metallische Werkstoffe in Pulverform. Zumindest einer der Härter enthält mindestens teilweise Kationen in gelöster Form wie sie auch Bestandteil des festen Werkstoffs in Pulverform in der Dispersion sind. Mit anderen Worten, die Dispersion und zumindest einer der Härter enthalten Kationen der gleichen Art. Bei den Kationen handelt es sich insbesondere um die Ionen erster Art, die Ionen zweiter Art und/oder die Ionen einer weiteren Art. Die Verwendung der gleichen Kationen für den Härter und den Werkstoff in Pulverform, ist es möglich die physikalischen und/oder chemischen Eigenschaften des Grundmaterials, das heißt der keramischen und/oder glaskeramischen und/oder metallischen Werkstoffe in Pulverform nahezu konstant zu halten. Somit ist es möglich beispielsweise hochreine Grundmaterialien nicht unnötig zu verunreinigen.

In einer bevorzugten Ausführungsform umfasst die Dispersion mindestens ein Polysaccharid und/oder in einer ersten erfindungsgemäßen Alternative mindestens ein Hydrokolloid in Form eines Alginats, insbesondere eines Ammonium-Alginats. In Abhängigkeit des ausgewählten Alginats können die Eigenschaften der Dispersion gezielt beeinflusst werden. Darüber hinaus können auch Dispersionen verwendet werden, die zwei unterschiedliche Alginate aufweisen. Hierdurch können insbesondere die rheologischen Eigenschaften und die Gel-Bildung gezielt beeinflusst werden.

In einer zweiten erfindungsgemäßen Alternative umfasst die Dispersion mindestens ein Polyacrylat. Polyacrylate sind in einer breiten Palette ab Werk erhältlich. Entsprechend besteht durch die gezielte Auswahl eines Polyacrylats ein vergleichsweise großer Spielraum bei der Beeinflussung der Viskosität der Dispersion sowie der Geleigenschaften. Als Polyacrylate können zum Beispiel reine Polyacrylsäuren verwendet werden. Insbesondere können SYNTRAN^{®} Polyacrylate, wie zum Beispiel SYNTRAN^{®}8220, SYNTRAN^{®}8550-N, SYNTRAN^{®}8740 eingesetzt werden.

Darüber hinaus können vorteilhafte Eigenschaften der Dispersion auch durch eine Kombination von Alginat und Polyacrylat erreicht werden. Dadurch kann insbesondere die Reaktionskinetik der Dispersion beeinflusst werden. Beispielsweise kann auf diese Weise eine niedrige Viskosität der Dispersion bei unveränderter Gelstärke erreicht werden.

Alternativ kann die Dispersion auch einen natürlichen oder synthetischen Polyelektrolyten und/oder mindestens ein Polysaccharid und/oder mindestens ein Hydrokolloid aus der Gruppe bestehend aus Pektin, Galaktomannan, Karrageen, Dextran, Gellan, Scleroglucan, Chitosan, wasserlöslichen Polyphosphazenen wie poly(bis(4-Carboxyphenoxy)Phosphazen), Polyaminosäuren, aufweisen. Die Verwendung von Polyelektrolyten, Polysacchariden und/oder Hydrokolloiden können im Allgemeinen als gesundheitlich unbedenklich angesehen werden. Zudem können diese Materialien auf einfache und günstige Art und Weise bereitgestellt werden. Durch die Kombination unterschiedlich dotierter und/oder zusammengesetzter Polyelektrolyten, Polysaccharide und/oder Hydrokolloide können die physikalischen und/oder chemischen Eigenschaften der Dispersion sowie die des Rohkörpers eingestellt werden. So bewirkt die Zugabe eines Polyelektrolyten oder Polysaccharids oder Hydrokolloids einerseits eine Veränderung der rheologischen Eigenschaften der Dispersion, andererseits verändern sich mit Art und Menge des Polyelektrolyten oder Polysaccharid oder Hydrokolloid die Eigenschaften des Rohkörpers.

Gemäß einer bevorzugten Weiterbildung umfasst die Dispersion mindestens zwei unterschiedliche Polyacrylate. Dadurch kann beispielsweise eine möglichst geringe Viskosität der Dispersion sowie eine möglichst hohe Festigkeit des daraus erzeugten Rohkörpers erreicht werden.

Es wird ferner ein nicht-erfindungsgemäßer Formkörper angegeben. Der Formkörper ist bevorzugt mit einem generativen Herstellungsverfahren, und besonders bevorzugt mit dem hier beschriebenen Verfahren, herstellbar. Das heißt, sämtliche für das Verfahren offenbarten Merkmale sind auch für den Formkörper offenbart und umgekehrt.

Der Formkörper ist vorzugsweise als dentale Prothese ausgebildet. Insbesondere ist der Formkörper als Brücke, als Brückengerüst, als Inlay, als Onlay, als Krone, als Implantat, als Abutment und/oder als Veneer ausgebildet, Der Formkörper enthält als Material eine Keramik, eine Glaskeramik und/oder ein Pulvermetall oder besteht aus einer Kombination dieser Materialien. Eine physikalische Eigenschaft und/oder eine chemische Eigenschaft des Formkörpers variiert lokal. Mit anderen Worten, der Formkörper weist in unterschiedlichen Bereichen entlang des Volumens des Formkörpers unterschiedliche physikalische und/oder chemische Eigenschaften auf. Beispielsweise kann die Farbe des Formkörpers lokal variieren.

Es wird zudem ein nicht-erfindungsgemäßes System zur generativen Herstellung eines Formkörpers angegeben. Das System ist bevorzugt dazu eingerichtet, ein hier beschriebenes Verfahren zur Herstellung eines Formkörpers, insbesondere eines hier beschriebenen Formkörpers, durchzuführen. Das heißt, sämtliche für das Verfahren und den Formkörper offenbarte Merkmale sind auch für das System offenbart und umgekehrt.

Das System zur generativen Herstellung eines Formkörpers umfasst entsprechend einen Spender und/oder einen Drucker zum Auftragen einer Dispersion und einer Vielzahl von unterschiedlichen Härtern. Die Dispersion weist mindestens einen Binder auf. Ferner weist die Dispersion Keramikpartikel und/oder Glaskeramikpartikel und/oder Pulvermetallpartikel auf. Bevorzugt ist der Drucker dazu eingerichtet, die jeweilige Menge der unterschiedlichen Härter ortsabhängig einzustellen. Ein relativer Anteil der Härter zueinander kann dann ortsabhängig mittels des Druckers gesteuert werden.

Beispielsweise umfasst das System zumindest die Dispersion und ferner wenigstens zwei unterschiedliche Härter. Die unterschiedlichen Härter können beispielsweise Ionen einer unterschiedlichen Art, wie beispielsweise Ionen einer ersten Art und Ionen einer zweiten Art, in unterschiedlichen Mengenverhältnissen aufweisen.

### Detaillierte Beschreibung bevorzugter Ausführunqsbeispiele

Nachfolgend ist ein Ausführungsbeispiel eines hier beschriebenen Verfahrens zur generativen Herstellung eines Formkörpers sowie eines hier beschriebenen Systems zur generativen Herstellung eines Formkörpers näher erläutert.

In einem ersten Verfahrensschritt wird eine Schicht einer Dispersion auf einen Hilfsträger aufgebracht. In einem zweiten Verfahrensschritt werden auf die Schicht der Dispersion Härter aufgebracht. Bei dem vorliegenden Ausführungsbeispiel wird ein Härter einer ersten Art und ein Härter einer zweiten Art verwendet. Beispielsweise enthält der Härter der ersten Art eine andere Materialzusammensetzung, insbesondere Ionen einer anderen Art, als der Härter der zweiten Art. Beispielsweise kann der Härter der ersten Art Titan und der Härter der zweiten Art Chrom umfassen. Alternative können die Härter der ersten und zweiten Art jeweils Mangan, Eisen, Kobalt, Nickel, Kupfer, Zink, Aluminium, Strontium, Yttrium, Zirkonium, Niob, Lanthan, Cer, Praseodym, Terbium, Erbium oder Ytterbium umfassen. Die sich voneinander unterscheidenden Arten von Härtern können entsprechend der gewünschten Farbgebung und/oder anderer physikalischer und/oder chemischer Eigenschaften des zu erzeugenden Rohkörpers gewählt werden.

Die Dispersion kann Aluminiumoxid als Rohstoff enthalten, beispielsweise kalziniertes Aluminiumoxid der Firma Baikowski (HP DBM). Ferner kann die Dispersion Alginat enthalten. Zur Bereitstellung der Dispersion können 24 Stunden vor der Verwendung der Dispersion in dem Verfahren eine Aluminiumoxid-Suspension (beispielsweise mit 56 Vol% Aluminiumoxid) und eine Alginat-Lösung (beispielsweise mit 2-3 Gew% Alginat) angesetzt worden sein. Die Aluminiumoxid-Suspension und die Alginat-Lösung können zu der Dispersion kombiniert werden. Die Alginat-Konzentration in der finalen Dispersion beträgt beispielsweise 0,4-0,75 Gew%, typischerweise 0,5 Gew%. Die Dispersion kann ferner 0,1 Gew% Ammoniumcitrat enthalten. Das Aufbringen der Schicht der Dispersion, der Härter der ersten Art und der Härter der zweiten Art erfolgt mittels eines Systems, welches einen Spender und einen Drucker umfasst. Der Spender enthält das Material der Dispersion und ist dazu eingerichtet, die Dispersion auf den Hilfsträger beziehungsweise auf in vorhergehenden Verfahrensschritten aufgebrachte Schichten der Dispersion aufzubringen.

Gemäß einer Ausführungsform kann der Hilfsträger nach jedem Auftragsschritt abgesenkt werden. Das Absenken hat ein Nachfließen der Dispersion aus dem Spender zur Folge, wobei die Dispersion über den Rohkörper fließt, während dieser um die abgesenkte Höhe in die Dispersion eintaucht. Alternativ kann der Spender in Form eines Druckkopfs bereitgestellt sein, welcher die Dispersion auf den Hilfsträger beziehungsweise auf den Rohkörper aufträgt.

Der Drucker ist dazu eingerichtet, eine einstellbare Menge des Härters erster Art und/oder des Härters zweiter Art auf die Schicht der Dispersion aufzudrucken. Hierfür kann der Drucker unterschiedliche Bereiche, wie zum Beispiel Kartuschen, aufweisen, in denen der Härter erster Art und der Härter zweiter Art eingebracht sind.

Die durch den Drucker bereitgestellte Tropfengröße beträgt beispielsweise 10 pl, wodurch die Positionierung der einzelnen Härter präzise eingestellt werden kann. Beispielsweise beträgt eine Schichtdicke des Härters erster Art und/oder des Härters zweiter Art 10 µm wenn eine Schichtdicke der Schicht der Dispersion 100 µm beträgt. Dies entspricht beispielsweise einer Menge des Härters erster und/oder zweiter Art von deutlich weniger als 1 µl/cm² der Schicht der Dispersion.

Die vorstehend beschriebenen Verfahrensschritte werden mehrmals wiederholt, wodurch ein Rohkörper bereitgestellt werden kann. Der Rohkörper enthält mehrere Schichten der Dispersion, zwischen denen jeweils Schichten des Härters erster Art und/oder des Härters zweiter Art angeordnet sind (im Folgenden auch "Schichtstapel"). Die jeweilige Menge des Härters erster Art und des Härters zweiter Art ist mittels des Druckers in den unterschiedlichen Schichten ortsabhängig verändert worden, wodurch der relative Anteil des Härters erster Art zu dem Härter zweiter Art lateral entlang der Schichten (xy-Ebene) und vertikal entlang des Schichtstapels (z-Richtung) variieren kann.

Zur Erhöhung der Festigkeit des Rohkörpers kann dieser nach seiner Fertigstellung zunächst gespült werden, beispielsweise mit Wasser, und anschließend mit einer Salzlösung nachbehandelt werden. Hierdurch kann der Rohkörper zusätzlich verfestigt werden.

In einem weiteren Verfahrensschritt kann der Rohkörper getrocknet und abgebindert werden. Beim Abbindern (auch Entbinderung genannt) wird der Rohkörper einer chemischen Behandlung und/oder einer Wärmebehandlung unterzogen, wodurch eventuell vorhandene Bindemittel, die beispielsweise in der Dispersion, dem Härter erster Art und/oder dem Härter zweiter Art enthalten sind, aus dem Rohkörper entfernt werden. Die Bindemittel werden durch das Abbindern pyrolysiert, vergast oder verbrannt. Das Trocknen und Abbindern kann beispielsweise in einem gemeinsamen Verfahrensschritt erfolgen.

Der Rohkörper wird anschließend gesintert. Das Sintern eines zum Beispiel aus Aluminiumoxid (Al₂O₃) vom Typ Baikowski HP DBM hergestellten Rohkörpers kann bei einer Sintertemperatur von 1600 °C erfolgen. Beispielsweise wird der Rohkörper für eine Stunde bei einer Heizrate von 10 °C/min gesintert. Hierdurch kann ein Formkörper mit einer Dichte von beispielsweise wenigstens 3,8 g/cm³ bis höchstens 4,0 g/cm³, bevorzugt wenigstens 3,90 g/cm³ und höchstens 3,98 g/cm³, bereitgestellt werden. Die Dichte des Formkörpers entspricht bevorzugt der beim Sintern erreichten Sinterdichte. Mit den verwendeten Sinter-Parametern ist es möglich, einen Formkörper mit einer Biegefestigkeit von 440 MPa bereitzustellen, der der ISO-Norm 6872 entspricht. Beispielsweise beträgt ein Schrumpf des Rohkörpers beim Sintern in jede Raumrichtung wenigstens 15 % und höchstens 30 %. Insbesondere kann ein lateraler linearer Schrumpf 18% und ein vertikaler Schrumpf 21% betragen. Um das Schrumpfen des Rohkörpers beim Sintern zu berücksichtigen, kann der Rohkörper mit größeren Abmessungen bereitgestellt werden.

Ein Formkörper wird bevorzugt mit dem zuvor beschriebenen Verfahren hergestellt. Der Formkörper enthält erste Bereiche, zweite Bereiche und dritte Bereiche, die jeweils unterschiedliche Konzentrationen beziehungsweise relative Anteile des Materials der Dispersion, des Härters erster Art und/oder des Härters zweiter Art enthalten. Die zweiten Bereiche und die dritten Bereiche können eine höhere Konzentration des Härters erster Art beziehungsweise des Härters zweiter Art als die ersten Bereiche aufweisen. Die ersten Bereiche, die zweiten Bereiche und die dritten Bereiche können durch eine ortsabhängige Einstellung des relativen Verhältnisses des Härters erster Art und des Härters zweiter Art zueinander entstanden sein. Der Härter erster Art und/oder der Härter zweiter Art kann innerhalb des Rohkörpers diffundieren und Ausscheidungen bilden, wodurch die unterschiedlichen Bereiche entstehen.

In den unterschiedlichen Bereichen weist der Formkörper unterschiedliche physikalische und/oder chemische Eigenschaften auf. Beispielsweise ist eine optische Eigenschaft des Formkörpers im ersten Bereich unterschiedlich von einer optischen Eigenschaft des Formkörpers im zweiten Bereich und/oder im dritten Bereich. Als optische Eigenschaft kommen beispielsweise die Transparenz, die Farbe und/oder die Opazität des Formkörpers in Betracht. Insbesondere wenn der Formkörper eine dentale Prothese ist, kann durch die Verwendung unterschiedlicher Härter, die die optischen Eigenschaften auf unterschiedliche Weise beeinflussen, die natürliche Variation der Farbe von Zähnen nachgeahmt werden, wodurch der dentalen Prothese ein natürliches Erscheinungsbild verliehen werden kann.

## Patentansprüche

1. Verfahren zur generativen Herstellung eines dentalen Formkörpers für die Zahnrestauration und/oder als Zahnersatz, aufweisend die folgenden Schritte:
a) Bereitstellen zumindest einer Schicht einer Dispersion, insbesondere einer wässrigen Dispersion, wobei die Dispersion mindestens einen Binder aufweist, und wobei die Dispersion Keramikpartikel und/oder Glaskeramikpartikel und/oder Pulvermetallpartikel aufweist;
b) stellenweises Aufbringen von Härtern auf die Schicht der Dispersion zur Aktivierung des mindestens einen Binders zur stellenweisen Verfestigung der Schicht der Dispersion, wodurch ein Rohkörper erhalten wird, insbesondere ein Grünkörper, wobei mindestens zwei Härter mit voneinander unterschiedlicher Materialzusammensetzung verwendet werden, wobei mindestens einer der Härter Ionen zumindest einer ersten Art enthält;
c) Sintern des Rohkörpers zu dem dentalen Formkörper
**dadurch gekennzeichnet, dass**
die Dispersion zum Einstellen der rheologischen und Gel bildenden Eigenschaften mindestens ein Hydrokolloid in Form eines Alginats, insbesondere eines Ammonium-Alginats, umfasst und/oder
dass die Dispersion mindestens ein Polyacrylat umfasst.

2. Verfahren nach Anspruch 1, **dadurch gekennzeichnet, dass** der mindestens eine Binder und die Keramikpartikel und/oder Glaskeramikpartikel und/oder Pulvermetallpartikel in der Dispersion homogen verteilt sind.

3. Verfahren nach Anspruch 1 oder 2, **dadurch gekennzeichnet, dass** ein relativer Anteil der Härter zueinander ortsabhängig gesteuert wird.

4. Verfahren nach einem der vorhergehenden Ansprüche, **dadurch gekennzeichnet, dass** mindestens einer der Härter Ionen zumindest einer zweiten Art enthält, wobei die Ionen zweiter Art während des Sinterns in Schritt b) zumindest teilweise in dem Kristallgitter des Rohkörpers in fester Lösung gelöst werden und die Ionen zweiter Art lokal zumindest eine physikalische Eigenschaft und/oder zumindest eine chemische Eigenschaft des Rohkörpers verändern.

5. Verfahren nach dem vorhergehenden Anspruch, **dadurch gekennzeichnet, dass** die zumindest eine physikalische Eigenschaft eine optische Eigenschaft des Formkörpers ist und/oder die zumindest eine chemische Eigenschaft eine katalytische Eigenschaft des Formkörpers ist.

6. Verfahren nach einem der vorhergehenden Ansprüche, **dadurch gekennzeichnet, dass** mindestens einer der Härter Ionen zumindest einer zweiten Art enthält, wobei die Ionen zweiter Art während des Sinterns in Schritt b) zumindest teilweise in dem Kristallgitter des Rohkörpers in fester Lösung gelöst werden und die für das Sintern in Schritt b) verwendete Sintertemperatur durch die Ionen zweiter Art reduziert wird.

7. Verfahren nach einem der vorhergehenden Ansprüche, **dadurch gekennzeichnet, dass** mindestens einer der Härter Ionen einer ersten Art und/oder Ionen einer zweiten Art enthält, wobei die Ionen der ersten Art und/oder die Ionen der zweiten Art während des Sinterns in Schritt b) Ausscheidungen im Gefüge der Kristallstruktur des Rohkörpers bilden.

8. Verfahren nach einem der vorhergehenden Ansprüche, **dadurch gekennzeichnet, dass** die Ionen erster Art und/oder die Ionen zweiter Art Kationen mit einer Wertigkeit von wenigstens drei sind.

9. Verfahren nach einem der vorherigen Ansprüche, **dadurch gekennzeichnet, dass** die Dispersion keramische und/oder glaskeramische und/oder metallische Werkstoffe in Pulverform umfasst, wobei mindestens einer der Härter ebenfalls mindestens teilweise Kationen in gelöster Form wie sie auch Bestandteil des festen Werkstoffs in Pulverform in der Dispersion sind umfasst.

10. Verfahren nach einem der vorhergehenden Ansprüche, **dadurch gekennzeichnet, dass** die Dispersion mindestens zwei unterschiedliche Polyacrylate umfasst.

## Claims

1. Method for generatively producing a dental moulding for dental restoration and/or as a dental prosthesis, having the following steps:
a) providing at least one layer of a dispersion, in particular an aqueous dispersion, wherein the dispersion has at least one binder, and wherein the dispersion has ceramic particles and/or glass ceramic particles and/or powder metal particles;
b) applying hardeners to the layer of the dispersion in places in order to activate the at least one binder in order to harden the layer of the dispersion in places, whereby a rough blank is obtained, in particular a green body, wherein at least two hardeners having a different material composition from each other are used, wherein at least one of the hardeners contains ions of at least a first type;
c) sintering the rough blank to form the dental moulding,
**characterised in that**
the dispersion comprises at least one hydrocolloid in the form of an alginate, in particular an ammonium alginate, for adjusting the rheological and gel-forming properties and/or
**in that** the dispersion comprises at least one polyacrylate.

2. Method according to claim 1, **characterised in that** the at least one binder and the ceramic particles and/or glass ceramic particles and/or powder metal particles are homogeneously distributed in the dispersion.

3. Method according to claim 1 or 2, **characterised in that** a relative proportion of the hardeners to each other is controlled in a location-dependent manner.

4. Method according to any of the preceding claims, **characterised in that** at least one of the hardeners contains ions of at least a second type, wherein the ions of the second type are at least partially dissolved in a solid solution in the crystal matrix of the rough blank during sintering in step b), and the ions of the second type locally modify at least a physical property and/or a chemical property of the rough blank.

5. Method according to the preceding claim, **characterised in that** the at least one physical property is an optical property of the moulding and/or the at least one chemical property is a catalytic property of the moulding.

6. Method according to any of the preceding claims, **characterised in that** at least one of the hardeners contains ions of at least a second type, wherein the ions of the second type are at least partially dissolved in a solid solution in the crystal matrix of the rough blank during sintering in step b), and the sintering temperature used for sintering in step b) is reduced by the ions of the second type.

7. Method according to any of the preceding claims, **characterised in that** at least one of the hardeners contains ions of a first type and/or ions of a second type, wherein the ions of the first type and/or the ions of the second type form precipitants in the microstructure of the crystal structure of the rough blank during sintering in step b).

8. Method according to any of the preceding claims, **characterised in that** the ions of the first type and/or the ions of the second type are cations having a valence of at least three.

9. Method according to any of the preceding claims, **characterised in that** the dispersion comprises ceramic and/or glass ceramic and/or metal materials in powder form, wherein at least one of the hardeners also comprises, at least partially, cations in dissolved form as the cations are also a component of the solid material in powder form in the dispersion.

10. Method according to any of the preceding claims, **characterised in that** the dispersion comprises at least two different polyacrylates.

## Revendications

1. Procédé de fabrication générative d'un corps moulé dentaire pour la restauration dentaire et/ou comme prothèse dentaire, présentant les étapes suivantes :
a) fournir au moins une couche d'une dispersion, en particulier une dispersion aqueuse, dans lequel la dispersion présente au moins un liant, et dans lequel la dispersion présente des particules de céramique et/ou particules de céramique de verre et/ou particules de métal pulvérulent ;
b) appliquer par endroit des agents durcisseurs sur la couche de dispersion pour l'activation d'au moins un liant pour la consolidation par endroits de la couche de dispersion, ce qui permet d'obtenir un corps brut, en particulier un corps vert, dans lequel au moins deux agents durcisseurs sont utilisés avec une composition de matériau différente l'une de l'autre, dans lequel au moins un des agents durcisseurs contient des ions au moins d'un premier type ;
c) fritter le corps brut pour former le corps moulé dentaire
**caractérisé en ce que**
la dispersion pour le réglage des propriétés rhéologiques et formant le gel comporte au moins un hydrocolloïde sous la forme d'un alginate, en particulier d'un alginate d'ammonium, et/ou
que la dispersion comporte au moins un polyacrylate.

2. Procédé selon la revendication 1, **caractérisé en ce que** l'au moins un liant et les particules de céramique et/ou particules de céramique de verre et/ou particules de métal pulvérulent sont répartis de manière homogène dans la dispersion.

3. Procédé selon la revendication 1 ou 2, **caractérisé en ce qu'**une part relative des agents durcisseurs est commandée en fonction de l'endroit l'un par rapport à l'autre.

4. Procédé selon l'une quelconque des revendications précédentes, **caractérisé en ce qu'**au moins un des agents durcisseurs contient des ions au moins d'un second type, dans lequel les ions du second type sont dissous pendant le frittage dans l'étape b) au moins partiellement dans le réseau cristallin du corps brut en solution solide et les ions du second type modifient localement au moins une propriété physique et/ou au moins une propriété chimique du corps brut.

5. Procédé selon la revendication précédente, **caractérisé en ce que** l'au moins une propriété physique est une propriété optique du corps moulé et/ou l'au moins une propriété chimique est une propriété catalytique du corps moulé.

6. Procédé selon l'une quelconque des revendications précédentes, **caractérisé en ce qu'**au moins un des agents durcisseurs contient des ions au moins d'un second type, dans lequel les ions du second type sont dissous pendant le frittage dans l'étape b) au moins partiellement dans le réseau cristallin du corps brut en solution solide et la température de frittage utilisée pour le frittage dans l'étape b) est réduite par les ions du second type.

7. Procédé selon l'une quelconque des revendications précédentes, **caractérisé en ce qu'**au moins un des agents durcisseurs contient des ions d'un premier type et/ou des ions d'un second type, dans lequel les ions du premier type et/ou les ions du second type forment pendant le frittage dans l'étape b) des dépôts dans l'assemblage de la structure cristalline du corps brut.

8. Procédé selon l'une quelconque des revendications précédentes, **caractérisé en ce que** les ions du premier type et/ou les ions du second type sont des cations avec une valence d'au moins trois.

9. Procédé selon l'une quelconque des revendications précédentes, **caractérisé en ce que** la dispersion comporte des matériaux céramiques et/ou céramiques de verre et/ou métalliques sous la forme pulvérulente, dans lequel au moins un des agents durcisseurs comporte aussi au moins partiellement des cations sous la forme dissoute comme ils font également partie du matériau solide sous la forme pulvérulente dans la dispersion.

10. Procédé selon l'une quelconque des revendications précédentes, **caractérisé en ce que** la dispersion comporte au moins deux polyacrylates différents.
